# EUROPEAN PATENT APPLICATION

(11) **EP 1 219 312 A2**
(43) Date of publication of application: **03.07.2002**
(21) Application number: 01126368.8
(22) Date of filing: 07.11.2001
(51) Int. Cl.: A61M 5/145

(54) **Portable apparatus for infusing drugs**

(30) Priority: 29.12.2000 IT TO001224
(71) Applicant: CANE' S.r.l., 10090 Rivoli (TO) (IT)
(72) Inventor: Cane', Mario, 10095 Collegno (To) (IT)
(74) Representative: Robba, Pierpaolo

(57) **Abstract**

A portable apparatus for infusing drugs comprises a syringe (111) connectable to a needle stably inserted in the patient and a pusher (108) having a rectilinear motion and being moved by a threaded shaft (107), said pusher co-operating with an end of the groove (113) of the syringe plunger for the programmed and controlled advance of the plunger itself. The threaded shaft (107) and the rod (113) are coaxially mounted in a seat (110) realised in the body (101) of the apparatus, and the terminal part of the threaded shaft (107), when the syringe is mounted, is received into an axial cavity of the rod (113).

## Description

The object of the present invention is a portable apparatus for infusing drugs, for instance insulin, of the type comprising a syringe that injects the drug through a cannula connected to a needle stably inserted in the patient, and means for the programmed and controlled advance of the syringe plunger, said means consisting of a pusher having a rectilinear motion and being moved by a threaded shaft that Is driven by a battery-operated electric motor.

Devices of this type are well known and an example is described in US-A-4.191.187. In said device, the pusher acts on the end of the plunger rod and the syringe is mounted parallel to the threaded shaft, said shaft being outside of the body or container housing the mechanical and electronic driving and regulating members. This arrangement presents the disadvantage that the dimensions are relatively high and that the push is misaligned with respect to the rod, with the risk of jamming.

Accordingly, a first object of the invention is that of providing an apparatus having limited dimensions and that does not require a misaligned push on the rod.

In order to reduce the dimensions, the Applicant has already proposed some devices wherein the rod can be separated from the plunger after that the syringe has been filled, and wherein the pusher is made to act on the plunger by means of an adaptor. However, said devices present some disadvantages in connection with the intervention on the rod, like the possibility of drug leakage, the difficulty in adjusting and mounting and a high cost of the syringe. Besides, the risk exists that the contact between the plunger and the connecting members with the pusher might be lost, with a consequent trigger for the phenomenon known as free flow that can cause the supply of a drug overdosage to the patient.

Therefore it is a second object of the invention to provide an infusion apparatus that, besides presenting limited dimensions and avoiding the misaligned push on the rod, does not present any risk of triggering the free flow phenomenon.

These objects are obtained by the fact that the syringe and the threaded shaft with the pusher are coaxially arranged in a seat inside the body of the apparatus and that the rod presents an axial cavity which houses, when the syringe is mounted, a terminal part of the threaded shaft extending beyond the pusher, said pusher being coaxially mounted on the threaded shaft.

Advantageously, the rod presents a hollow head able to engage on the pusher for preventing the rod from getting separated from the pusher.

For a better understanding, reference is made to the hereby attached drawings, wherein:
- Figure 1 is an exploded view of a first embodiment of the invention;
- Figure 2 is an axial sectional view of the syringe;
- Figure 3 is a cross-sectional view of the guide slot of the pusher; and
- Figure 4 is a partially exploded and partially cut-away sectional view of a second embodiment of the invention.

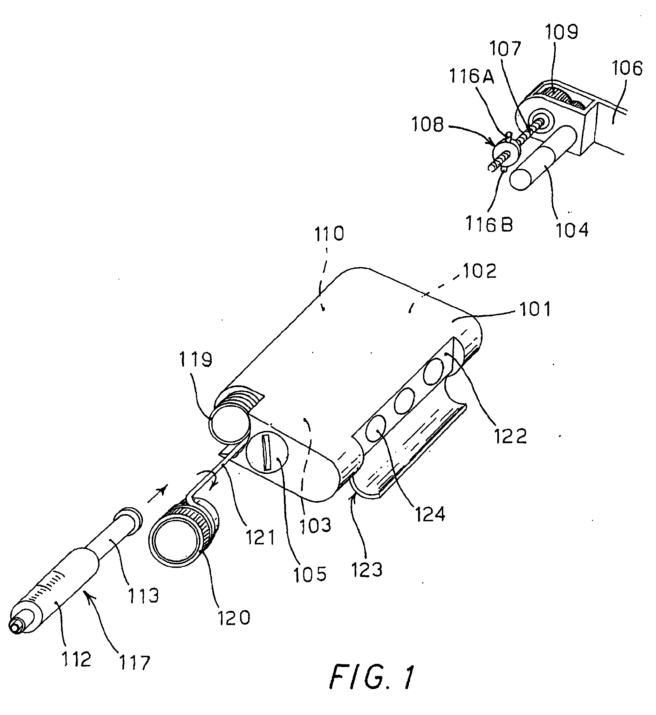

In both embodiments, homologous parts are indicated with the same reference numbers, preceded by the figure 1 and respectively 2.

With reference to the Figures 1-3, the apparatus according to the invention comprises a body 101, Inside of which seats 102 and 103 are realised for the motor 104 and for the relative power supply batteries (not shown). A plug 105 closes the seat 103. The terminal wall 106 of the body 101 opposed to the wall provided with the plug 105 carries the motor 104, the threaded shaft 107 with the pusher 108, and the mechanisms 109 for transmitting motion from the motor 104 to the threaded shaft 107. The control and programming electronic circuits, that are not concerned by the invention and have not been shown, are arranged inside the body 101 as well.

The threaded shaft 107 is housed in a longitudinal through cavity 110, laterally realised in the body 101, which also houses the syringe 111. Said syringe 111 Is Introduced through a sleeve 119 provided at the end of the cavity 110 opposed to the end where the threaded shaft 107 is introduced. The threaded shaft 107 and the syringe 111 are coaxially arranged in the cavity 110. A threaded lock ring 120 can be slipped onto the syringe 111 and engages with the externally threaded sleeve 119. The ring 120 is advantageously constrained to the body 101, for instance by means of a wire or flexible cable 121 realised in plastic or metal.

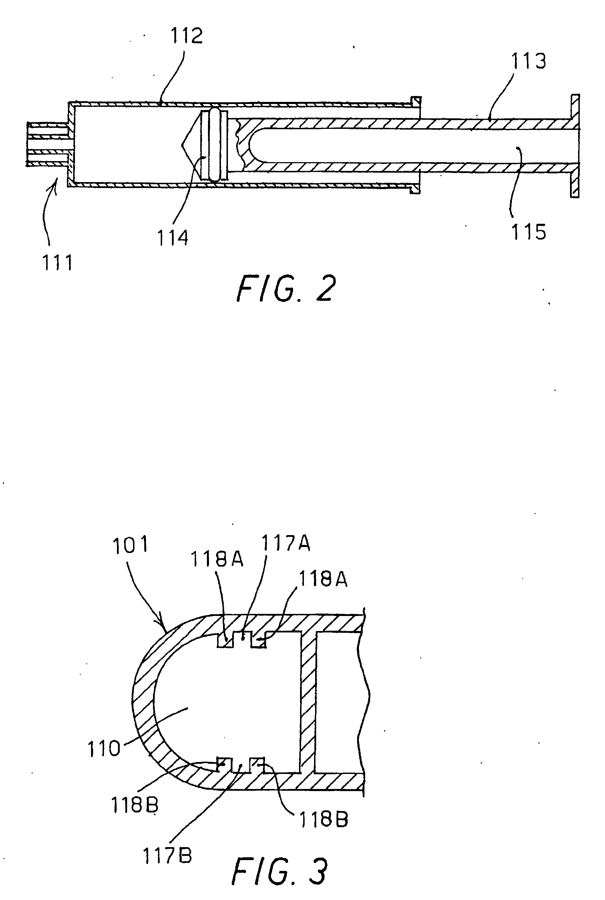

The syringe 111 comprises, as usual, a body 112 and a rod 113 connected with the plunger 114 (Figure 2), but the rod 113 presents an axial cavity 115 so that it can be slipped onto the threaded shaft 107 when the syringe 111 is mounted in the cavity 110.

The pusher 108 is for instance a discoidal element that is coaxially mounted on the threaded shaft 107 and that is conventionally moved in rectilinear motion by said shaft 107. The pusher 108 presents a pair of diametrically opposed pins 116A,116B, having for instance a vertical axis, able to engage in guide slots 117A,117B (Figure 3) defined by pairs of ribs 118A,118B respectively realised on the vault and on the base of the cavity 110 in the region corresponding to the travel of the pusher 108. In this way, a rotation of the pusher 108 is avoided.

Finally, on the side opposed to the cavity 110, the body 101 presents a groove 122, closed by a flap door 123, wherein the push-button controls 124 of the apparatus are arranged.

Advantageously, with the embodiment described with reference to Figure 1, it will be possible to obtain a sealed apparatus.

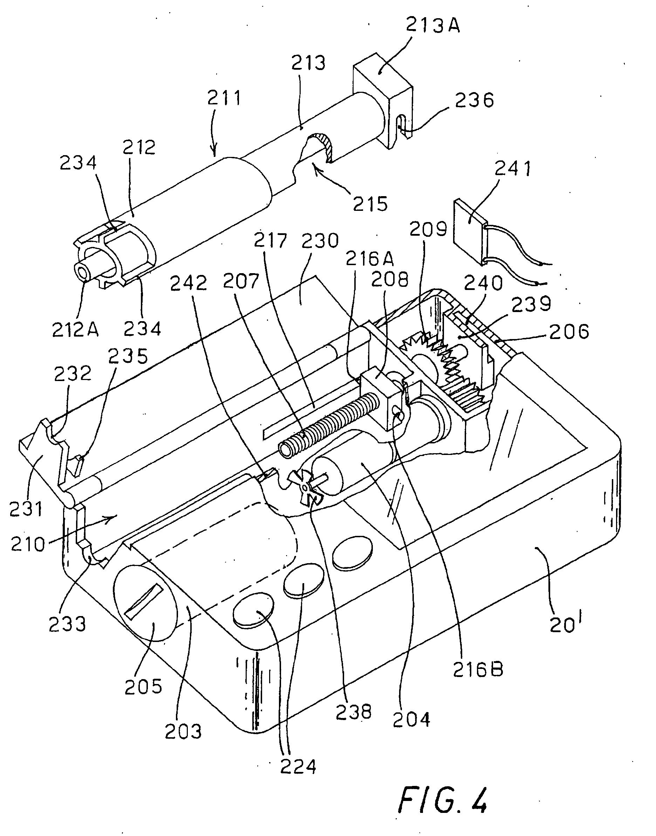

In the embodiment of Figure 4, the threaded shaft 207 and the syringe 211 are housed in a space 210 which is open at its upper and frontal parts. The space 210 Is provided with a flap door 230 having a closure extension 231 of the frontal opening. The extension 231 presents on its tip a semicircular groove 232 defining, with a complementary groove 233 on the frontal wall of the body 201, a passage for the conical point or cone 212A of the syringe 211. A bulkhead 242 separates the seat 210 from the rest of the Internal space of the body 201.

The body 212 of the syringe 211 presents, in proximity of the conical point or cone 212A, a reduced radius region with radial tongues 234 engaging with locks 235 realised on the lid 230 and/or on the walls of the space 210 for preventing the syringe from rotating. The rod 213 is hollow, like in the embodiment of Figures 1-3, and has a substantially parallelepiped-shaped hollow head 213A that can be put on the pusher 208 where it is held thanks to the engagement between a pair of grooves 236 (only one of which is visible in the Figure) of opposed side walls of the head 213A and pins 216A,216B laterally projecting from the pusher 208. In this way, the pusher is prevented from getting separated from the rod head, which could originate the free flow. In order to allow the insertion of the threaded shaft 207 into the cavity 215, the rod 213 will be open at its bottom. The end of the pins 216 engages in grooves 217 realised on the side walls of the seat 210.

The motor 204, that could be a stepping motor, is provided with an encoder 238 for sensing the angular position that allows also to recognise motor stalls due to the occlusion of the cannula connecting the syringe with the needle inserted in the patient's body. As an alternative or in addition to the use of the encoder 238, the threaded shaft 207 can rest on a deformable bulkhead 239 that is connected with the transversal closure wall 206 of the body 201 and delimits with said wall a seat 240 for a pressure sensor 241. An increase of pressure on the sensor 241 is an index of an occlusion in the cannula supplying the drug to the needle. The sensor 241 is advantageously calibrated so that it closes a contact and activates an alarm when a threshold stress lower than the stall stress of the motor 204 is reached.

It is clear that what has been described is given as a not limiting example and that changes and modifications are possible without departing from the field of protection of the invention. For instance, the arrangement of the push buttons of Figure 4 could be analogous to that of Figure 1.

## Claims

1. Portable apparatus for infusing drugs, comprising a syringe (111; 211) that injects the drug through a cannula connected to a needle stably inserted in the patient, and a pusher (108; 208) having a rectilinear motion and engaging on a threaded shaft (107; 207) that is driven by a battery-operated electric motor (104; 204) and co-operates with an end of a rod (113; 213) connected with a plunger (114) of the syringe (111; 211) for the programmed and controlled advance of the plunger (114) itself, **characterised by** the fact that the syringe (111; 211) and the threaded shaft (107; 207) with the pusher (108; 208) are coaxially arranged in a seat (110; 210) internal to a body (101; 201) of the apparatus, and said rod (113; 213) presents an axial cavity (115; 215) wherein it is received, when the syringe (111; 211) is mounted, a terminal part of the threaded shaft (107; 207) which extends beyond the pusher (108; 208), said pusher being coaxially mounted on the threaded shaft (107; 207).

2. Apparatus according to claim 1, **characterised by** the fact that said pusher (108; 208) is constituted by a disk or plate element presenting a pair of projecting in opposite directions pins (116A,116B; 216A,216B) that engage with respective guide slots (117A,117B; 217) realised in said seat (110; 210), for a length substantially corresponding to the travel of the pusher (108; 208).

3. Apparatus according to claim 1 or 2, **characterised by** the fact of presenting a lateral recess (122), closed by a flap door (123), wherein push-buttons (124) are arranged for controlling and regulating the apparatus.

4. Apparatus according to anyone of the preceding claims, **characterised by** the fact that said seat (110) is a longitudinal cavity that opens in opposed terminal walls of the body (101) of the apparatus and Is connected, from the side where the syringe (111) is introduced, to an externally threaded sleeve (119), a threaded lock ring (120) engaging on the sleeve for blocking the syringe in the seat (110).

5. Apparatus according to claim 4, **characterised by** the fact that the lock ring (120) is provided with a cable (121) for fastening said ring to the body (101) of the apparatus.

6. Apparatus according to anyone of the claims 1-3, **characterised by** the fact that the rod (213) of the syringe (211) presents a hollow head (213A) able to engage on the pusher (208).

7. Apparatus according to claim 6, **characterised by** the fact that said hollow head (213A) is parallelepiped-shaped and presents, on two parallel side walls, a pair of grooves (236) wherein the pins (216) projecting from the pusher (208) engage, for preventing the rod (213) from getting separated from the pusher (208).

8. Apparatus according to claim 6 or 7, **characterised by** the fact that the axial cavity (215) of the rod (213) presents a longitudinal split for the passage of the threaded shaft (207).

9. Apparatus according to anyone of the claims from 6 to 8, **characterised by** the fact that said seat (210) is a space open at its upper and frontal parts, with a closure lid (230) presenting a frontal wall (231) shaped so that it defines, with the frontal wall of the body (201) of the apparatus, a passage (232,233) for a conical point or cone (212A) of the syringe (211).

10. Apparatus according to anyone of the claims from 6 to 9, **characterised by** the fact that the syringe (211) presents a body (212) with a terminal part, adjacent to the conical point or cone (212A), equipped with tongues (234) engaging with locks (235) provided on the walls of the seat (210) for preventing the syringe (211) from rotating.

11. Apparatus according to anyone of the claims from 6 to 10, **characterised by** the fact that it comprises means (238,241) for recognising a stall in the advance of the pusher (208) due to an interruption of the drug coming out of the syringe.

12. Apparatus according to claim 11, **characterised by** the fact that the recognising means comprise an encoder (238) associated to the motor (204) and/or a pressure sensor (241) mounted in the back part of the threaded shaft (208) and calibrated so that it provides a signal alarm when a threshold stress lower than the stall stress of the motor (204) is reached.
